# EUROPEAN PATENT SPECIFICATION

(11) **EP 1 805 306 B1**
(45) Date of publication and mention of the grant of the patent: **07.01.2009**
(21) Application number: 05795375.4
(22) Date of filing: 20.10.2005
(51) Int. Cl.: C12N 15/11, C12N 15/87, C12N 15/88

(54) **VEHICLE TO TRANSPORT A DNA-MODIFYING ENZYME TO A GENOME**
TRÄGER FÜR DEN TRANSPORT EINES DNA-MODIFIZIERENDEN ENZYMS ZU EINEM GENOM
VEHICULE DESTINE AU TRANSPORT D'UNE ENZYME MODIFICATRICE D'ADN VERS UN GENOME

(30) Priority: 21.10.2004 NL 1027311; 04.11.2004 NL 1027417; 10.11.2004 NL 1027479
(43) Date of publication of application: 11.07.2007
(73) Proprietor: Synvolux IP B.V., 9713 GX Groningen (NL)
(72) Inventor: RUITERS, Marcel, Herman, Josef, NL-9728 WN Groningen (NL)
(74) Representative: Kupecz, Arpad
(86) International application number: PCT/NL2005/000753
(87) International publication number: WO 2006/043810

(56) References cited:
- EP-A- 0 755 924
- WO-A-03/010308
- WO-A-03/033701
- WO-A-20/04050885
- STEENSEL VAN B ET AL: "Identification of in vivo DNA targets of chromatin proteins using tethered Dam methyltransferase" NATURE BIOTECHNOLOGY, NATURE PUBLISHING, US, vol. 18, no. 4, April 2000 (2000-04), pages 424-428, XP002187507 ISSN: 1087-0156

## Description

The present invention relates to a vehicle to transport a DNA-modifying enzyme to a desired site in a genome. Further the invention relates to the application of the vehicle of the invention comprising a SAINT-molecule.

It is known from the state of the art that cancer and metabolic diseases are in many cases caused by undesired regulation of specific genes. In molecular biological research on this type of diseases often DNA-modifying enzymes (such as restriction enzymes, methylases and demethylases etc.) are used. These enzymes are specific in such a manner that in the genome of humans, animals, plants, bacteria and viruses they have a certain specificity for recognising a particular nucleotide sequence. Specific nucleotide sequences of that sort are usually found several times, even sometimes thousands of times per genome.

The above described technology is known from the article by Paul S. Lovett and Michael G Bramucci, "Evidence of a non-random base sequence in a Bacillus pumilus plasmid: EcoR1 endonuclease digestion of pPL576" Journal of Bacteriology, July 1975, pg 377/379 Vol 123 (1). Furthermore this technology is known from the Handbook "Molecular cloning: a laboratory manual", ed. T. Maniatis, published by Cold Spring Harbor.

WO 2004/050885 A discloses a method for suppressing the expression of a selected apoptosis-related gene in a cell.

WO 03/010308 A relates to a method of suppressing the expression of a selected endogenous gene in a eukaryotic cell.

STEENSEL VAN B ET AL: "Identification of in vivo DNA targets of chromatin proteins using tethered Dam methyltransferase" NATURE BIOTECHNOLOGY, NATURE PUBLISHING, US, vol. 18, no. 4, April 2000 (2000-04), pages 424-428, XP002187507 ISSN: 1087-0156 discloses identification of in vivo DNA targets of chromatin proteins using tethered Dam methyltransferase.

EP-A-0 755 924 discloses transport vehicles for macromolecules.

The aim of the present invention is to generate an enzyme that is specific for one place in the genome.

In particular the aim of the present invention is to provide the possibility that the desired site in the genome can be chosen very specific. In case the chosen site in the genome (a regulating nucleotide sequence of a gene) is responsible for a disease, the present invention provides the possibility of shutting off this gene by targeting a specific enzyme to the regulating nucleotide sequence of the gene. The present invention provides this opportunity by means of the measures described in the characterising part of claim 1.

Further advantageous embodiments are described in the dependent conclusion 3.

According to a further aspect the invention relates to the application of a vehicle, which comprises a SAINT-molecule, to transport an active compound to a cell.

SAINT-molecules are already known, for example indicated as transport vehicle, and have been extensively described in the European Patent, publication number EP-0 755 924-B1 and the US Patent US-5,853,694.

According to a preferred embodiment, the enzyme is coupled to the sequence recogniser by a spacer molecule. Thereby a spatial separation is generated between the sequence recognizing entity and the enzyme, as a result of which the enzyme activity is not inhibited, when the vehicle is bound to the genome. In this way the enzyme is able to perform its function in the way the enzyme is normally used to.

In order to obtain a desired specificity for a favoured place in the genome, preferable triple-helix forming units or oligonucleotides intercalating in the minor grooves of the DNA are used. That sort of sequence recognizers have a structure which conforms itself to the desired place in the genome only. As a result, the specificity becomes very high and, when these sequence recognisers are synthesised based on a known structure, they will be made unique to one place in the genome only. The technology is commonly know to an person skilled in the field and it is described in more detail in the article of F. Sanger et al, "Use of DNA polymerase I primed by a synthetic oligonucleotide to determine the sequence in phage f1 DNA" Proc. Nat. Acad. Sci. USA vol. 70, no. 4. pg 1209-1213, April 1973. Furthermore, this technique is described in the Handbook "Molecular cloning: a laboratory manual", ed. T. Maniatis, published by Cold Spring Harbor.

Therefore it is preferred that the sequence recognizer has a DNA or PNA structure which is specific to the desired place in the genome. Such structures can be synthesised relatively easy.

According to another preferred embodiment it is preferred that the desired enzyme has a low Km. In this way the enzyme will perform its action only if it is bound to the DNA. In case the enzyme is not fixed to the DNA it will not be able to perform its activity. According to a preferred embodiment, it is preferred that the enzyme contains a so called "conformational switch". Such a conformational switch takes care that the enzyme will not be active without the DNA being bound on the desired place. The reason for this is the otherwise inappropriately folded protein structure.

In order to facilitate a good transport of DNA modifying enzymes to the cell, preferably said DNA modifying enzymes will be combined with the vehicle which is a SAINT-molecule or a combination of SAINT-molecules. Optionally, additional compounds may be present with this.

It is particularly preferred that the SAINT-molecule interacts through hydrogen bonding. The SAINT-molecule enwraps the sequence recognize and the enzyme that is covalently bound to the sequence recognizer. The SAINT-molecule as such is not bound to the DNA modifying enzymes but it just interacts therewith through hydrogen bonding. This hydrogen bonding becomes looser when the complex contacts the cell content after fusion with the cell membrane.

The DNA sequence, which is based on a 21 meric (this means a chain of 21 units) oligonucleotide, PNA or another entity, is specific for the human genome. A 21 mer appears only once. Notably, 4 to the power of 21 is much larger then the size of the human genome. As soon as TFO binds, the enzyme will fold around the DNA and performs its function. The oligonucleotide, however, will stay attached to the DNA, thereby assuring the enzyme can perform its function only once. At the moment the cell divides, the DNA will by "cleaned" from these uncommon proteins, the enzyme will be removed and be degraded through the usual ubiquitin route.

## Claims

1. A vehicle for the transport of a DNA-modifying enzyme/molecule to the nucleus, which is **characterised in that** the DNA-modifying enzyme is combined with a Saint-molecule or a combination of several entities of it.

2. The vehicle according to claim 1, **characterised in that** the Saint-molecule is bound to the DNA-modifying enzyme by means of a hydrogen bonding.

3. In vitro Use of a SAINT-molecule in combination with the DNA-modifying enzyme according to claim 1 and 2, for the transport of the enzyme to the cell and to the nucleus thereof.

## Patentansprüche

1. Träger für den Transport eines DNA-modizifierenden Enzyms/Moleküls zum Zellkern, **dadurch gekennzeichnet, dass** das DNA-modifizierende Enzym mit einem SAINT-Molekül oder einer Kombination aus mehreren Entitäten von diesem kombiniert ist.

2. Träger nach Anspruch 1, **dadurch gekennzeichnet, dass** das SAINT-Molekül an das DNA-modizifierende Enzym mitteln einer Wasserstoffbindung gebunden ist.

3. *In vitro* Verwendung eines SAINT-Moleküls in Kombination mit dem DNA-modizifierenden Enzym nach Anspruch 1 und 2, für den Transport des Enzyms zur Zelle und zu deren Zellkern.

## Revendications

1. Véhicule pour le transport vers le noyau d'une molécule/enzyme de modification de l'ADN, qui est **caractérisé en ce que** l'enzyme de modification de l'ADN est combinée avec une molécule-SAINT ou une combinaison de plusieurs entités de celle-ci.

2. Véhicule selon la revendication 1, **caractérisé en ce que** la molécule-SAINT est liée à l'enzyme de modification de l'ADN par l'intermédiaire d'une liaison hydrogène

3. Utilisation *in vitro* d'une molécule-SAINT en combinaison avec l'enzyme de modification de l'ADN selon les revendications 1 et 2, pour le transport de l'enzyme vers la cellule et vers son noyau.
